# EUROPEAN PATENT APPLICATION

(11) **EP 4 825 970 A1**
(43) Date of publication of application: **21.10.2026**
(21) Application number: 26172282.1
(22) Date of filing: 14.04.2026
(51) Int. Cl.: C12N 15/113, C12N 9/22

(54) **ENDOGENOUS INTRONIC RNA FOR TIGHT CONTROL OF CAS/CRISPR-MEDIATED GENE EDITING IN HUMAN CELLS**

(30) Priority: 14.04.2025 PT 2025120205; 16.06.2025 PT 2025120343; 10.04.2026 PT 2026121320
(71) Applicant: Egas Moniz - Cooperativa de Ensino Superior, CRL, 2829-511 Caparica (PT)
(72) Inventor: SANTIAGO DE LEMOS CARNEIRO, André João, Eixo (PT); E MELO CABRAL BARRETO, Vasco Temudo, Caparica (PT)
(74) Representative: Pereira da Cruz, Joao

(57) **Abstract**

The present disclosure refers to a CRISPR/Cas-based gene editing system for conditional activation in human cells, characterized in that the system comprises: a Cas protein or a functionally equivalent variant thereof; and at least one conditionally activatable guide RNA (gRNA), wherein the gRNA is designed to be activated by hybridization with an endogenous intronic RNA molecule in the human cell, thereby enabling targeted DNA cleavage by the Cas protein. It further discloses an *in vitro* process for activating a guide RNA (gRNA) in a CRISPR/Cas-based gene editing system in human cells, characterized in that the process comprises the following steps: i) providing a conditionally activatable gRNA; ii) exposing the gRNA to an endogenous intronic RNA molecule in the human cell; iii) hybridizing the gRNA with the intronic RNA molecule, thereby activating the gRNA and enabling Cas-mediated DNA cleavage at a target genomic locus, and its use in *in vitro* targeted gene editing, functional genomics, and therapeutic genome engineering in human cells.

## Description

### FIELD OF THE INVENTION

The present disclosure relates to the fields of genetic engineering, molecular biology, and genome editing. More specifically, it relates to CRISPR/Cas9-based gene editing systems employing conditionally activatable guide RNAs (gRNAs) designed to respond to endogenous intronic RNA molecules in human cells. The technology concerns the design and application of engineered conditional gRNAs that remain inactive until hybridization with specific intron-derived RNA triggers, thereby enabling controlled activation of Cas9-mediated DNA cleavage. This approach provides a strategy for achieving cell-type-specific and context-dependent genome editing by exploiting naturally occurring intronic transcripts as endogenous regulatory signals. The invention is particularly relevant for applications in targeted gene editing, functional genomics, and therapeutic genome engineering in human cells.

### PRIOR ART

CRISPR-Cas9 is a powerful gene-editing technique, but its low efficiency and specificity limit its applicability.

Although cgRNAs have previously been described in the prior art, they have predominantly relied on messenger RNA (mRNA) or microRNA (miRNA) triggers. These approaches show variable success rates and face significant limitations in broader applicability across different contexts; for example, suitable miRNAs may not be expressed in all cell types, and mRNA-based triggers have demonstrated low effectiveness due to physical constraints, particularly transcript length.

A cgRNA is a guide RNA that contains modified secondary structures that regulate its gene-editing activity by disrupting domains critical for its function. In some modalities, the cgRNA remains inactive until the presence of a specific trigger RNA restores the functional conformation. In other designs, the cgRNA is active by default and becomes inactivated upon binding to the trigger.

Until now, introns were largely overlooked as cgRNA triggers for several reasons. These include the complex secondary structures formed by introns during splicing, uncertainties about whether splicing occurs post-transcriptionally or co-transcriptionally (and, in the latter case, whether effective hybridization would be possible), and concerns that the rapid degradation of introns might prevent effective hybridization. These concerns may have contributed to the belief that intronic RNA would be unavailable or unreliable for hybridization-based activation or deactivation.

Despite the wide adoption of CRISPR technology for gene editing, including in clinical applications, important challenges remain, including unwanted editing events. In a broad sense, such unwanted editing includes both editing at unintended genomic regions, commonly referred to as off-target editing, and editing at the intended genomic locus in cells, tissues, or biological contexts in which editing is not intended.

Editing at unintended genomic regions, commonly referred to as off-target editing, may arise because the protospacer recognized by the gRNA is short and can tolerate mismatches, thereby enabling recognition of partially homologous sites elsewhere in the genome. Moreover, even editing at the correct target locus may be undesirable when it occurs in the wrong cell type or under the wrong biological conditions. The present invention mitigates these problems by making guide activity dependent on a specific endogenous intronic RNA trigger.

Furthermore, several approaches have been described, including the use of bioinformatic tools to find the guides with the fewest off-targets, the use of the combined action of two Cas9 nickase mutant to produce the DSB, guided by a pair of guides that recognize nearby protospacers, engineered Cas9 proteins with improved specificity, and methods that bypass the generation of DSBs, namely the editing with base editors and prime-editing.

These efforts have not solved the off-target problem completely. For instance, the improvements in specificity provided by the "high-fidelity" Cas9 variants are relatively small and specificity seems to be negatively correlated with efficiency. Likewise, base editors have been shown to induce unwanted off-target mutations. Besides the engineering of the protein component of the RNP complex, the crRNA has also been modified to improve the control of the activity of Cas9 and decrease the number of off-targets.

Various technologies have been developed to conditionally activate/deactivate CRISPR/Cas9 in the presence of a specific stimuli, each with distinct advantages and limitations. These can be broadly categorized into non-RNA-based triggers (such as drug-inducible and light-activated systems) and RNA-based triggers (such as mRNA, miRNA), some of which are below identified.

Drug-Inducible CRISPR Systems (non-RNA-based) such as patent document US11643672B2, depend on exogenous drugs that may cause immunogenicity, toxicity, or off-target effects (especially in vivo), and it can exhibit systemic leakage where even low inducer doses partially activate the system, limiting precise control.

Light-Activated (optogenetic) CRISPR Systems (non-RNA-based) such as patent document US 11214779B2, require specialized laser or LED equipment and is limited by poor light penetration into deep tissues, restricting its scalability and use in whole organisms.

RNA-based systems are limited by the need for short mRNA triggers with efficiency dropping significantly for transcripts longer than ^{~}400 nucleotides, does not address off-target effects due to the widespread distribution of mRNA in the cell, and requires identifying suitable mRNAs with strong cell- or tissue-specific expression for reliable activation.

Conditional Cas9 expression by mRNA regulation has the limitation of a leaky expression still causing moderate level of gene editing in current state of the art methods, requiring robust design.

The solution of the present disclosure is an intron RNA trigger intended to innovatively overcome such issues.

The present approach enables endogenous control of gene editing by allowing conditional activation based on the presence of specific cellular intronic RNA already present in cells, such as disease-associated markers, developmental signals, or cell-type-specific transcripts, thereby enabling selective targeting of cells expressing particular gene products.

In addition, the present disclosure offers the potential for nuclear spatial control of gene editing because intron RNAs generally remain in close proximity to the gene that encodes them, providing precise nuclear localization that could enable highly localized editing and reduce unintended modifications at distant genomic sites.

The present invention has a broad applicability, as many eukaryotic genes contain introns with tissue- or stage-specific splicing patterns that can be exploited to restrict CRISPR activity to predefined cell populations.

Importantly, since approximately 98% of monogenic genetic diseases are caused by mutations in genes containing both exons and introns, compared with about 2% associated with miRNA genes, the present technology greatly expands the potential gene pool for regulated gene editing.

Furthermore, it can be applied to genes with long mRNA transcripts provided they contain short introns, substantially increasing the range of genes that can be used to control gene editing compared with current state-of-the-art technologies.

### GENERAL DESCRIPTION

Genome editing is a molecular technique that enables precise modifications of DNA sequences in living cells. It uses targeted endonucleases, *i.e.* enzymes that introduce double-strand breaks (DSBs) at specific genomic locations. These breaks trigger cellular repair mechanisms, either through non-homologous end joining (NHEJ), which can create insertions or deletions, or homology-directed repair (HDR), allowing precise sequence modifications.

Among genome-editing tools, the CRISPR/Cas system has become the most widely used due to its simplicity, efficiency, and versatility. The CRISPR/Cas system uses a guide RNA (crRNA) to direct the Cas9 endonuclease to a complementary DNA sequence, where it induces a DSB.

In an embodiment, variants such as Cas9 nickase, Cas12, and Cas13 provide expanded capabilities, including targeting single-stranded DNA and RNA. CRISPR-based techniques are adaptable for therapeutic applications, functional genomics, and agricultural improvements.

The present disclosure relates to a method for regulating CRISPR/Cas gene-editing activity using a new class of activating molecules for conditional guide RNAs (cgRNAs), namely intronic RNA. A cgRNA responsive to intronic RNA is here referred to as an incgRNA. In addition, this disclosure also describes a new cgRNA design that is an improvement of a single-guide RNA described in the literature (10.1089/crispr.2020.0029) that was optimized and adapted for the crRNA + tracrRNA system.

In one embodiment, the CRISPR/Cas-based gene editing system enabling conditional activation in human cells of the present disclosure is used for precise, stimulus-responsive regulation of gene expression for applications in targeted therapeutics, controlled cell reprogramming, disease modeling, and adaptive biomanufacturing.

In one embodiment, the system comprises a spacer, which in the context of the present disclosure is a spacer region binding to a complementary sequence (the protospacer) in the genome, allowing the endonuclease to be targeted to the genomic region of choice.

The terms "incgRNA" and "intcgRNA" are equivalent and are used interchangeably in the present application, which correspond to conditionally activatable guide RNA (gRNA).

In a preferred embodiment, the present disclosure refers to a CRISPR/Cas-based gene editing system for conditional activation in human cells, characterized in that the system comprises: a Cas protein or a functionally equivalent variant thereof; and at least one conditionally activatable guide RNA (gRNA), wherein the gRNA is designed to be activated by hybridization with an endogenous intronic RNA molecule in the human cell, said intronic RNA molecule having a length of less than 300 nucleotides, preferably less than 250, even preferably less than 210 nucleotides, thereby enabling targeted DNA cleavage by said Cas protein.

In a further embodiment, the disclosure refers to a system wherein an endogenous intronic RNA molecule in the human cell is SEQ ID No. 1 or a sequence with 90% homology thereto, the target region being SEQ. ID No. 2 or a sequence with 90% homology thereto.

In a further embodiment, the disclosure refers to a system wherein Cas is Cas3, Cas9, Cas9 nickase, Cas12 or Cas13.

In a further embodiment, the disclosure refers to a system wherein the conditionally activatable guide RNA comprises SEQ ID No. 8.

In a further embodiment, the disclosure refers to a system, wherein the conditionally activatable guide RNA is SEQ. ID No. 4, SEQ ID No. 5 or SEQ ID No. 6 or a sequence with 90% homology thereto, preferably 95% homology, even preferably 99% homology.

In a further embodiment, the disclosure refers to a system, wherein the conditionally activatable guide RNA is SEQ ID No. 5 or a sequence with 90% homology thereto, preferably 95% homology, even preferably 99% homology.

In a further embodiment, the disclosure refers to a system for use in regulation of gene expression for applications in targeted therapeutics, controlled cell reprogramming, disease modelling and adaptive bioengineering, in particular to minimize off-target effects at distant genomic sites.

In a further embodiment, the disclosure refers to an *in vitro* process for activating a guide RNA (gRNA) in a CRISPR/Cas-based gene editing system in human cells, characterized in that the process comprises the following steps:
i) providing a conditionally activatable gRNA according to the preferred embodiment;
ii) exposing the gRNA to an endogenous intronic RNA molecule in the human cell;
iii) hybridizing the gRNA with the intronic RNA molecule, thereby activating the gRNA and enabling Cas-mediated DNA cleavage at a target genomic locus.

In a further embodiment, the disclosure refers to process wherein the endogenous intronic RNA molecule in the human cell is SEQ ID No. 1 or a sequence with 90% homology thereto, preferably 95% homology, even more preferably 99% homology, the target region being SEQ. ID No. 2 or a sequence with 90% homology thereto, preferably 95% homology, even more preferably 99% homology.

In a further embodiment, the disclosure refers to a process wherein Cas is Cas3, Cas9, Cas12 or Cas13.

In a further embodiment, the disclosure refers to the use of the system and process of the present disclosure *in in vitro* targeted gene editing, functional genomics, and therapeutic genome engineering in human cells.

### DESCRIPTION OF FIGURES

The following figures provide preferred embodiments for illustrating the disclosure and should not be seen as limiting the scope of invention.

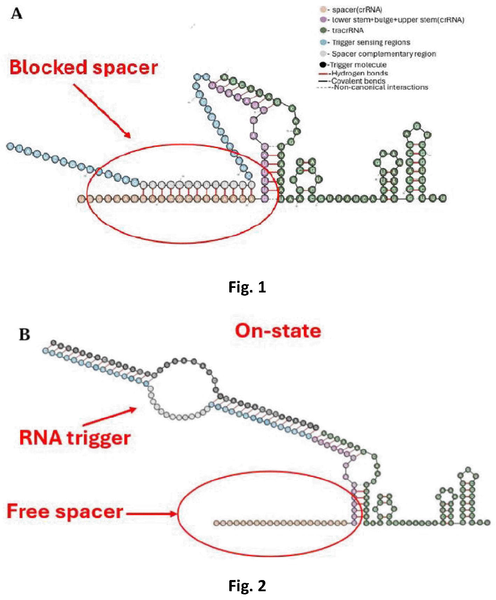

**Figure 1** is an example of an incgRNA in its off state. A double-stranded blocking region (a duplex) (an extension of the 3' end of the CRISPR RNA) creates a double-stranded region with the seed sequence that prevents target recognition. The blocking region (a 13-base-pair duplex in this example) is flanked by 15 nucleotides on each side that hybridize with the trigger intron.
**Figure 2** is an incgRNA in its on state after binding to the intron-derived RNA trigger. The hybridization event dismantles the double-stranded blocking region and releases the "spacer" region, allowing the gRNA to bind to the target DNA and enabling Cas9 to cleave the target DNA.

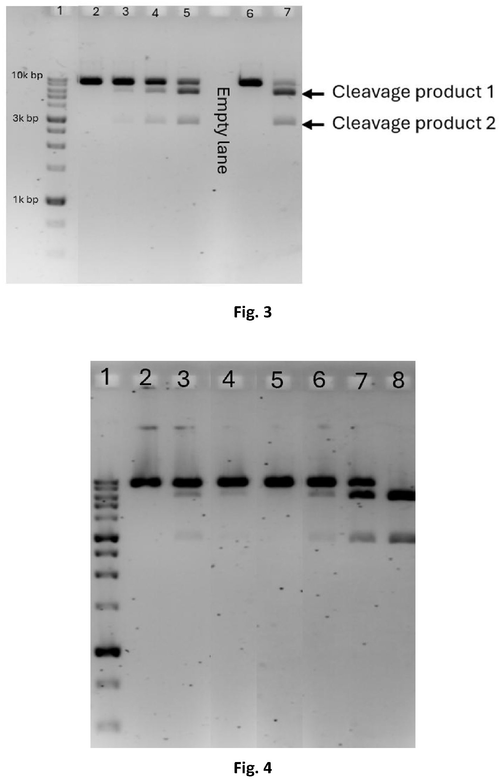

**Figure 3** is an in vitro cleavage assay to compare the 13-bp duplex incgRNA with the corresponding (same spacer) native guide. A linearized plasmid containing the protospacer was incubated for 127 minutes at 37°C in the presence of different ribonucleoproteins composed of Cas9 and different guide RNAs, with or without the trigger molecule. Unlike the standard (unmodified) gRNA (lane 7), the incgRNA is not active in the absence of the trigger (lane 6) but becomes active in a dose-dependent manner in the presence of the trigger (lanes 3-5). Lanes: 1 DNA ladder. 2. Plasmid only. 3. Plasmid with incgRNA and trigger 1:1. 4. Plasmid with incgRNA and trigger 1:2. 5. Plasmid with incgRNA and trigger 1:6. 6. incgRNA only. 7. Plasmid with Native gRNA.
**Figure 4** is an in vitro cleavage assay examining the effect of spacer duplex length on incgRNA activity. The plasmid containing the protospacer was incubated with Cas9-guide RNA complexes at 37°C. The incgRNA variants with different spacer duplex lengths (11, 12, and 13 bp) were tested both in the absence and presence of the trigger molecule. Lanes: (1) DNA ladder, (2) plasmid only control, (3-5) incgRNA with increasing spacer duplex lengths (11-13 bp) in the absence trigger, (6-8) incgRNA with decreasing spacer duplex lengths (13-11 bp) with trigger present.

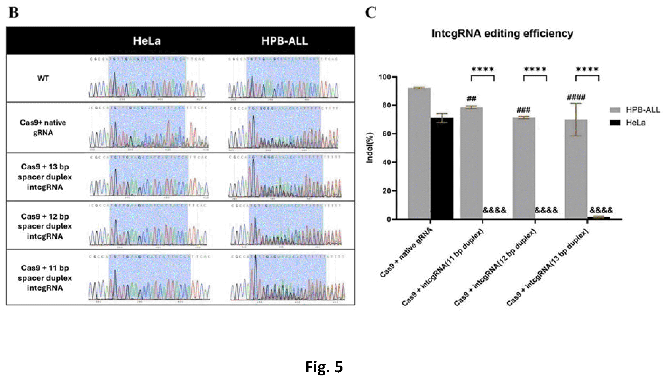

**Figure 5** shows that intcgRNA edits the target with an efficiency comparable to that of the conventional crRNA, but only in cells in which the target gene is markedly transcribed. B) Sequence alignments of the target region amplified from GFP+ cells 72 hours after electroporation with the RNP complexes and a GFP-encoding plasmid. C) Quantification of the editing events detected by Sanger sequencing using ICE. The data from B and C are representative of two independent experiments. Bars show mean ± SD (n = 3 independent biological replicates). Statistical significance was determined using two-way ANOVA followed by Šídák's multiple comparisons test. Symbols denote significance of comparisons: * indicates comparison between HPB-ALL and HeLa for the same experimental condition; # indicates comparison of each intcgRNA to native gRNA condition within HPB-ALL; & indicates comparison of each intcgRNA to native gRNA condition within HeLa. Symbol key: two symbols, p < 0.01; three symbols, p < 0.001; four symbols, p < 0.0001.

### DETAILED DESCRIPTION

The more general and advantageous configurations of the present invention are described in the Summary of the invention. Such configurations are detailed below in accordance with other advantageous and/or preferred embodiments of implementation of the present invention.

The use of conditional guide RNAs (cgRNAs) in the CRISPR-Cas9 system provides a critical layer of control over the activity of Cas9 by ensuring that target recognition and cleavage occur only in the presence of specific activating molecules or molecular conditions. This conditional activation mechanism provides an additional layer of control by restricting guide RNA activity to cellular contexts in which the activating intronic RNA is present, thereby enabling more selective and context-dependent genome editing. By coupling Cas9 activity to defined intracellular signals, cgRNAs enhance temporal and potentially spatial precision of genome editing, improving specificity and safety profiles, which is particularly relevant for therapeutic and high-fidelity applications where minimizing unintended genomic modifications is essential.

In a further embodiment, the present disclosure increases CRISPR-Cas9 specificity by using introns from the target gene as endogenous activators of CRISPR-Cas9 editing.

In a particular embodiment, the invention is not limited to a specific nucleotide composition, but instead encompasses any intron having a length of 300 nucleotides or less, as well as longer introns,, provided they retain the ability to selectively interact with the corresponding intcgRNA and promote its conformational activation, are considered suitable for use within the scope of the present invention, enabling binding to the intcgRNA and subsequent activation of gene editing mechanisms.

In a further embodiment, a sequence similar to intron 2 of the IL2RG gene is used, whose intronic RNA sequence is SEQ ID No. 1 (5'-3') and, when present in cells, binds to the intcgRNA, thereby unlocking it and activating gene editing (the region shown in bold corresponds to the portion of the intron that base pairs with the incgRNA):

guaugagaagggacgaggggaggggaugaagaagagguggguuggaucagagaccaagagag
aggguagcaagucucccagguaccccacuguuuucuccuggguaagucauaagucgguugaggggaga
ugaggcuaggcucuggauaucugcaguacccagauuggccccacuguuccucuuccuuccaaccuuucu
ccucuag.

SEQ. ID Nº 1 exists in significant amounts in HPB-ALL (human leukemia cell line derived from acute lymphoblastic leukemia (ALL)), having been used as a proof of concept to demonstrate that gene editing could be restricted exclusively to cells that produce a specific intron at significant levels (the bolded portion corresponds to the target sequence SEQ ID No. 2.

SEQ ID No. 3 (5'-3') refers to the IL2RG gene sequence, the target sequence at bold:

acagacagactacacccagggaatgaagagcaagcgccatgttgaagccatcattaccattcacatccctcttatt
cctgcagctgcccctgctgggagtggggctgaacacgacaattctgacgcccaatgggaatgaagacaccacagc
tggtgggaaatctgggactggagggggctggtgagaagggtggctgtgggaaggggccgtacagagatctggtg
cctgccactggccattacaatcatgtgggcagaattgaaaagtggagtgggaagggcaaggggagggttccctg
cctcacgctacttcttctttctttcttgtttgtttgtttctttctttcttttgaggcagggtctcactatgttgcctaggctg
gtctcaaactcctggcctctagtgatcctcctgcctcagcctttcaaagcaccaggattacagacatgagccaccgt
gcttggcctcctccttctgaccatcatttctctttccctccctgccttcattttctccccaatctagatttcttcctgacca
ctatgcccactgactccctcagtgtttccactctgcccctcccagaggttcagtgttttgtgttcaatgtcgagtacatg
aattgcacttggaacagcagctctgagccccagcctaccaacctcactctgcattattggtatgagaagggacgag
gggagggggatgaagaagaggtgggttggatcagagaccaagagagagggtagcaagtctcccaggtacccca
ctgttttctcctggggtaagtcataagtcggttgaggggagatgaggctaggctctggatatctgcagtacccagatt
ggccccactgttcctcttccttccaacctttctcctctaggtacaagaactcggataatgataaagtccagaagtgca
gccactatctattctctgaagaaatcacttctggctgtcagttgcaaaaaaaggagatccacctctaccaaacattt
gttgttcagctccaggacccacgggaacccaggagacaggccacacagatgctaaaactgcagaatctgggtaat
ttggaaagaaagggtcaagagaccagggatactgtgggacattggagtctacagagtagtgttcttttatcataag

ggtacatgggcagaaaagaggaggtaggggatcatgatgggaagggaggaggtattaggggcactaccttcagg
atcctgacttgtctaggccaggggaatgaccacatatgcacacatatctccagtgatcccctgggctccagagaac
ctaacacttcacaaactgagtgaatcccagctagaactgaactggaacaacagattcttgaaccactgtttggagc
acttggtgcagtaccggactgactgggaccacagctggactgtgagtgactagggacgtgaatgtagcagctaag
gccaagaaagtagggctaaaggattcaaccagacagatagaaggacctaatatcaagctcctgttctctgcctccc
agcttctctgctcacccctaccctccctcctccaactcctttccccctatttctccagtgagttttctttttttcttttct
tttctttctttctttcttttttttttttttgagacagagcctcactctgttgcccaggcttgagtgcagtggggcgatctt
gggctcactgcgacctctgtctccctggttcaagtgattctcctgcttcagcctcccaagtagctgggagcatgcaca
accatgcctggctaattttgtatttttagtaaagacagggttttgccatgttggtcaggctggtcttgaactcctgac
ctcaggtgatctgcccacctcggcctcccaaagtgctgggattacaggcgtgagccaccattcctgacaccagtga
gttttcattagggattccctacccatactcttcctgataccagatagacaagtaaacaaaaggaagccattaaggg
gatccagaggggaggcattagattcaagtcagtgaagggagcagtgtggcttgagtagtcaagagatgagagag
aaactgggcagtagcagagatgacactggtgggtgttcaggagtatgttttaattctcccttctctcatagacaccc
actttccctcatcctctttctcctcaaggaacaatcagtggattatagacataagttctccttgcctagtgtggatggg
cagaaacgctacacgtttcgtgttcggagccgctttaacccactctgtggaagtgctcagcattggagtgaatggag
ccacccaatccactgggggagcaatacttcaaaaggtaaaatgggcccacatgacccaatccatgagcccaacac
cccagcctttctaacaccactgtcttttgctccacttccctgtcactaaagcccctaaacttggtgccccatctctccac
actgtctaaccccaacctctagaaatcaaggtttttctgtgtagggttgggttagcgtgttgttagagtaggggagtg
gattgagaaggaggctgaggggtactcaagggggctatagaatgtataggatttccctgaagcattcctagagag
cctgcaaggtgaagatggctttggaaccagctggatctaggctgtgccacatactacctctttggccttggccacat
ccctaaactcttggattctgtttcctaagatgtaagatggaggtaattgttcctgcctcacaggagctgttgtgagga
ttaaacagagagtatgtctttagcgcggtgcctggcaccagtgcctggcatgtagtaggggcacaacaaatataag
gtccactttgcttttctttttctatagagaatcctttcctgtttgcattggaagccgtggttatctctgttggctccatgg
gattgattatcagccttctctgtgtgtatttctggctggaacggtgagatttggagaagcccagaaaaatgaggga
acggtagctgacaatagcagaggagggttttgcagggtctttaggagtaaaggatgagacagtaagtaatgagag
attacccaagagggtttggtgatggaaggaagccacaggcacagagaacacagaatcactttatttcatatggga
caactgggagaagggtgataaaaaagctttaacctatgtgctcctgctccctctttctcccctgtcaggacgatgccc
cgaattcccaccctgaagaacctagaggatcttgttactgaataccacgggaacttttcggtgagaacgctgtcata
agcatgctgcagtctatcaactgccaactgcctgccagcaagacagacagagtgtgggggtgggggcagagagg
agagggaaggaggccctgcactaactgtcaggatgtggccgaccaaatggggcatggactatacagagagagac
acacacagaagtgcagattatagattgaatgaggcagatggcaactggtattgggggcccaggagcctgtgtatc
ccttctgtaatcaattacagtggttgcagacatcatgagtactcctttggcacagagctcggtcttttacttcctgccc

ctaattgacccctgacctggacatatctgtctttaggcctggagtggtgtgtctaagggactggctgagagtctgcag
ccagactacagtgaacgactctgcctcgtcagtgagattcccccaaaggaggggcccttggggaggggcctggg
gcctccccatgcaaccagcatagcccctactgggcccccccatgttacaccctaaagcctgaaacctgaaccccaa
tcctctgacagaagaaccccagggtcctgtagccctaagtggtactaactttccttcattcaacccacctgcgtctca
tactcacctcaccccactgtggctgatttggaattttgtgcccccatgtaagcaccccttcatttggcattccccacttg
agaattacccttttgccccgaacatgtttttcttctccctcagtctggcccttccttttcgcaggattcttcctccctccct
ctttccctcccttcctctttccatctaccctccgattgttcctgaaccgatgagaaataaagtttctgttgataatcatc
aaaaa.

In a particular embodiment, an RNA-responsive conditional guide RNA (cgRNA) is disclosed. Two cell lines were electroporated with the cgRNA and a GFP-encoding plasmid. One of the cell lines expresses high levels of the activating intron, whereas the other expresses very low levels. Transfected cells (GFP-positive) were isolated, and CRISPR edits were inferred by Sanger sequencing.

The results in Figure 5 demonstrate high levels of gene editing by the activated guides in the HPB-ALL cell line, and undetectable editing with the best intcgRNA variants in the cell line where the intron is not significantly expressed., suggesting the first successful intron-mediated activation of cgRNA, which could help overcome the limitations associated with using mRNA or miRNA as activators.

In one embodiment, the cgRNA includes a blocking sequence that forms a double-stranded region (duplex region) with the spacer sequence, thereby preventing the spacer from binding to the target in the off state. The blocking sequence may be included as an extension of the crRNA at the 3' end and create a double-stranded region that includes the spacer seed region, as in the new cgRNA design used in our experiments, although other structural configurations known in the art may also be employed.

Notably, any of the various conditional guide RNA designs known in the art that successfully function with miRNA or mRNA triggers can be adapted to detect intronic RNA.

The sequences of the guide RNAs used in the non-limiting examples shown here are described in Table 1, presenting the sequences of the incgRNA and standard guide RNA targeting the IL2rg locus. All RNAs share an identical "spacer" sequence (underlined) that recognizes the same target site in the IL2rg gene, differing in their structural components. The incgRNAs incorporate additional nucleotides that form the double-stranded region that blocks the "spacer" and the regions that detect the trigger.

**Table 1**

| Oligo Name | Sequence |
|---|---|
| crRNA-incgRNA 13bp duplex | /AlTR1/UGGUAAUGAUGGCUUCAACAGUUU UAGAGCUAUGCUCAAUCUGGGUACUGCUG UUGAAGCCAUCGAGCCUAGCCUCAUC/AlTR2 / |
| crRNA-incgRNA 12bp duplex | /AlTR1/UGGUAAUGAUGGCUUCAACAGUUU UAGAGCUAUGCUCAAUCUGGGUACUGCUG UUGAAGCCAUGAGCCUAGCCUCAUC/AlTR2/ |
| crRNA-incgRNA 11bp duplex | /AlTR1/UGGUAAUGAUGGCUUCAACAGUUU UAGAGCUAUGCUCAAUCUGGGUACUGCUG UUGAAGCCAGAGCCUAGCCUCAUC/AlTR2/ |
| crRNA-native gRNA | /AlTR1/UGGUAAUGAUGGCUUCAACAGUUU UAGAGCUAUGCU/AlTR/ |

| | |
|---|---|
| AITR1 and AITR2 are protective elements of the sequence which improve hybridization. | |

### Examples

For intcgRNA design optimization a gene whose expression was intended to modulate editing activity was first selected. The introns of that gene were then analysed to identify candidate trigger regions for intcgRNA design. For the proof-of-concept, intron 2 of IL2RG was used as the input sequence for trigger selection, and the trigger-sensing regions of the intcgRNA were optimised using NUPACK. Candidate trigger sequences were additionally screened using blastn on the NCBI BLAST web server to minimise undesired sequence complementarity with the intcgRNA. To quantify indel frequency, genomic DNA was purified from sorted cells by resuspending the cell pellet in 200 µL of Q buffer. The suspension was then subjected to the following thermocycler program: 50ºC for 2 hours, followed by 95ºC for 15 minutes. CRISPR editing efficiency (indel%) was inferred from Sanger trace data using Synthego ICE and TIDE. The primers used to sequence the target region were IL2RGFw (5'-AGTCAAGGAAGAGGCATGGC) and IL2RGRv (5'-AGATCTCTGTACGGCCCCTT).

The incgRNA was first validated *in vitro* in a cleavage assay using an incgRNA design with a 13-bp duplex between the blocking sequence and a spacer. A mixture A containing 500 nM of standard (unmodified) gRNA or incgRNA (either one annealed to tracrRNA using the following procedure: RNA oligos were obtained dry (lyophilized) and resuspended in IDTE buffer (IDT) to a concentration of 100 µM. For annealing, equal volumes of oligos were mixed and diluted in Duplex Buffer (IDT; 14 µL of Duplex Buffer for every 2 µL of native crRNA/tracrRNA or intcrRNA/tracrRNA mixture), then incubated at 95 °C for 4 minutes. The samples were then gradually cooled to 25 °C at a rate of 1 °C/min. If necessary, they were further diluted to the desired concentration in Duplex Buffer), 250-2000 nM of trigger RNA molecule, 1 µL of RiboLock RNase Inhibitor (Thermo Fisher, United States of America, Cat. No E00382), and Duplex Buffer (Integrated DNA Technologies, United States of America, Cat. No #11-05-01-03) was added to create a final mixture volume of 11 µL. Mixture A was incubated at 37°C for 60 min. Next, 15 µL of a reaction mixture B containing 1X NEBuffer 3.1 (New England Biolabs, United States of America, Cat. No B7203), 500 nM Cas9 (Integrated DNA Technologies, United States of America, Cat. No M06505), 1.5 µL of linearized px330-mSA (1.7 µg, Addgene Plasmid #113096) plasmid with the spacer target sequence in RNase-Free Water (Nzytech, Portugal, Cat. No 7732-18-5) was added into mixture A and the resulting mixture was incubated at 37°C for 4 hours. The reaction mixture was sampled at different time points. After collection, 8 µL of Q buffer (0.1 mg/mL Proteinase K, 0.01 M Tris-HCl pH 8.3, 0.000144% SDS ref: DOI: 10.2144/02325pf01) and 1 µL of 0.5 M EDTA (Panreac AppliChem, Spain, Cat. No A4892) were added to each sample to digest DNA-bound Cas9 and inactivate Cas9, respectively. Each sample was also incubated at 65°C to guarantee further reaction inactivation.

The reaction mixture was then analyzed using 1.8-2%agarose gel electrophoresis. For tests where th ribonucleoproteins (RNPs) were assembled before trigger sensing, native gRNA/cgRNAs were incubated with Cas9 in duplex buffer with RiboLock RNase Inhibitor for 20 minutes before the trigger molecule was added to the mixture. RNPs were then incubated with the trigger molecule for 1 hour prior to being added to the mixture with the 1X NEBuffer 3.1(NEB) and linearized target plasmid. This assay revealed that the incgRNA is not active in the absence of the trigger and becomes active in a dose-dependent manner in the presence of the trigger molecule (FIG. 3).

To optimize the incgRNA design and performance, it was evaluated how spacer duplex length influences activity by engineering variants with 13, 12, and 11 base pair duplexes between the spacer and blocking sequences (Table 1). It was verified that longer duplexes more strongly sequestered the spacer sequence, providing tighter off-state control but reduced on-state activity when triggered, while shorter duplexes showed the opposite behavior.

The incgRNA variants were then validated in human cells by targeting the 4174-bp IL2RG in two distinct human immortalized cell lines: 1) HPB-ALL (a peripheral blood-derived cell line) that expresses the gene and consequently the 208-bp intron used as the incgRNA trigger; 2) HeLa (an epithelial cell line) that expresses the trigger in negligible amounts.

Cells were cultured at 37°C, 5% CO2. HPB-ALL cells were cultured in Roswell Park Memorial Institute medium (RPMI 1640, Biowest, France, Cat. No. L0500), supplemented with 10% heat-inactivated Fetal Bovine Serum (Biowest, France, Cat. No 51400), and Penicillin/Streptomycin (Gibco, United Kingdom, Cat. No 15140148) at 100 µ/mL of penicillin, and 100 µg/mL of streptomycin. HeLa cells were in Minimum Essential Medium (Biowest, France, Cat. No L0440), supplemented with 10% heat-inactivated Fetal Bovine Serum (Biowest) and Penicillin/Streptomycin (Gibco) at 100 µ/mL of penicillin, and 100 µg/mL of streptomycin. Transfection. All cell lines were transfected by electroporation. Briefly, a 1- 2 million cell pellet obtained was resuspended in 100 µL of 1 M eletroporation buffer (5 mM KCI, 15 mM MgCl2, 120 mM Na2HPO4/NaH2PO4 pH 7.2, 50 mM Mannitol and 0.05% PEG. 0.1 nmol of Cas9 (IDT), 0.12 nmol of gRNA (formed by annealing 0.12 nmol each of crRNA and 0.12 nmol of tracrRNA using the procedure described in Example 1, without addition of RNase Inhibitor) and a GFP-expressing plasmid were added to the reaction mix. The mix was transferred to an electroporation cuvette and electroporated in an Amaxa Nucleofector 2b (Lonza, Switzerland, Cat. No. AAB-1001) using the program X-001 for all cell lines; afterward, 200 µL of culture medium was added to the cuvette and transferred to cell culture plates. Cells were analyzed by flow cytometry 72 hours after nucleofection in Sony SH800Z Sorting Flow Cytometer. GFP+ cells were interpreted as cells that also integrated the RNPs.

Comparisons were drawn between a standard/native (unmodified) guide RNA, which yielded robust editing in both HPB-ALL and HeLa cells, and the three incgRNA variants (FIG. 5).

Among these variants, the 12-bp variant achieved superior performance in cellular experiments, exhibiting comparable editing efficiency to the standard gRNA in HPB-ALL cells while showing undetectable editing in HeLa cells by Sanger sequencing. While this 12-bp variant had shown slight editing activity *in vitro* in the off-state, its performance in the cellular context proved optimal.

These results confirm that introns can effectively activate a conditional guide RNA only in cells that express the target gene, offering a new layer of tissue or cell-type specificity without compromising editing efficiency. The present disclosure demonstrates for the first time that intron RNA can serve as a potent and selective "switch" to shift a blocked guide RNA from its "off" to its "on" state. This technology can be employed to achieve selective, tissue- or cell-specific gene editing, leading to safer therapeutic interventions and cleaner bioengineering. The incgRNA can be used alone or in combination with other approaches designed to reduce off-targets.

Besides the tissue and or cell specificity allowed by incRNA, the precise and unique spatial localization within the nucleus of the trigger molecule, which is found mostly next to the transcribed gene, can potentially be leveraged to minimize off-target effects at distant genomic sites, a major obstacle in the broader application of gene therapy, which other approaches mitigate only partially and none of the other conditional guides described in the literature addresses.

In an embodiment, activating Cas, preferably Cas9 only in the vicinity of the target gene would: 1) vastly increase the choice of guides, which is presently constrained by the off-target potential; 2) decrease the number of off-targets within the target cell. These two features would complement the already described and validated cell and tissue specificity. They would also make incgRNA the first of the approaches to improve specificity that circumscribes editing activity to the target gene by making it dependent on the specificity of the guide and the local levels of intronic RNA, which peak near the target gene.

As will be clear to one skilled in the art, the present invention should not be limited to the embodiments described herein, and a number of changes are possible which remain within the scope of the present invention.

The disclosure should not be seen in any way restricted to the embodiments described and a person with ordinary skill in the art will foresee many possibilities to modifications thereof.

The above-described embodiments are combinable.

The following claims further set out particular embodiments of the disclosure.

## Claims

1. A CRISPR/Cas-based gene editing system for conditional activation in human cells, **characterized in that** the system comprises:
a Cas protein or a functionally equivalent variant thereof; and
at least one conditionally activatable guide RNA (gRNA), wherein the gRNA is designed to be activated by hybridization with an endogenous intronic RNA molecule in the human cell, said intronic RNA molecule having a length of less than 300 nucleotides, preferably less than 250, even preferably less than 210 nucleotides, thereby enabling targeted DNA cleavage by said Cas protein.

2. The system of claim 1 wherein an endogenous intronic RNA molecule in the human cell is SEQ ID No. 1 or a sequence with 90% homology thereto, the target region being SEQ. ID No. 2 or a sequence with 90% homology thereto.

3. The system of any of the previous claims wherein Cas is Cas3, Cas9, Cas9 nickase, Cas12 or Cas13.

4. The system of any of the previous claims wherein the conditionally activatable guide RNA comprises SEQ ID No. 8.

5. The system of any of the previous claims, wherein the conditionally activatable guide RNA is SEQ. ID No. 4, SEQ ID No. 5 or SEQ ID No. 6 or a sequence with 90% homology thereto, preferably 95% homology, even preferably 99% homology.

6. The system according to the previous claim, wherein the conditionally activatable guide RNA is SEQ ID No. 5 or a sequence with 90% homology thereto, preferably 95% homology, even preferably 99% homology.

7. The system of any of the previous claims for use in regulation of gene expression for applications in targeted therapeutics, controlled cell reprogramming, disease modelling and adaptive bioengineering, in particular to minimize off-target effects at distant genomic sites.

8. An *in vitro* process for activating a guide RNA (gRNA) in a CRISPR/Cas-based gene editing system in human cells, **characterized in that** the process comprises the following steps:
i) providing a conditionally activatable gRNA according to claims 1-7;
ii) exposing the gRNA to an endogenous intronic RNA molecule in the human cell;
iii) hybridizing the gRNA with the intronic RNA molecule, thereby activating the gRNA and enabling Cas-mediated DNA cleavage at a target genomic locus.

9. The process of the previous claim wherein the endogenous intronic RNA molecule in the human cell is SEQ ID No. 1 or a sequence with 90% homology thereto, preferably 95% homology, even more preferably 99% homology, the target region being SEQ. ID No. 2 or a sequence with 90% homology thereto, preferably 95% homology, even more preferably 99% homology.

10. The process of the claims 8-9 wherein Cas is Cas3, Cas9, Cas12 or Cas13.

11. Use of the system of claims 1-7 in *in vitro* targeted gene editing, functional genomics, and therapeutic genome engineering in human cells.

12. Use of the process of claims 8 - 10 in *in vitro* targeted gene editing, functional genomics, and therapeutic genome engineering in human cells.
